# EUROPEAN PATENT APPLICATION

(11) **EP 4 154 816 A1**
(43) Date of publication of application: **29.03.2023**
(21) Application number: 21198784.7
(22) Date of filing: 24.09.2021
(51) Int. Cl.: A61B 5/369, A61B 5/386, A61B 5/256, A61B 5/291, A61B 5/00

(54) **DEVICE FOR PLACEMENT OF SKIN-CONTACTING ELECTRODES**

(71) Applicant: Oncomfort SA, 1300 Wavre (BE)
(72) Inventor: Toussaint, Clémence, Oteppe (BE); Huyghe, Mario, Desselgem (BE); Tesse, Julien, Auderghem (BE)
(74) Representative: De Clercq & Partners

(57) **Abstract**

Provided is a device (100) for placement of one or more skin-contacting electrodes on a subject (50) comprising a planar support (120) having: a longitudinal main body portion (140) and a side arm portion (170) joined at its near end (18) to the main body portion (140), wherein portion of the main body portion (140) contains a landing region (142, 142') for mechanical and electrical dismountable attachment of an electronic unit (300), the main body portion (140) contains a plurality of signal-conducting elements (148a to d) that are skin-contacting electrodes or mounts for dismountable attachment of skin-contacting electrodes.

## Description

### Field of the invention

The present invention is in the field of electrode placement, in particular a device for ease of placement of electrodes for transmission of electroencephalogram (EEG) signals and/or of other signals (e.g. electromyography (EMG), electrocardiogram (ECG), and/or for transcranial direct current stimulation (tDCS)) across a head of a subject.

### Background to the invention

Problems in the art of electrode placement on subject, in particular of electrodes for collection of electroencephalogram (EEG) signals and other signals from the scalp include ease and speed of application by the user (e.g. health care practitioner, subject, care person) signal quality, manufacturing costs, comfort, and hygiene. The presently provided devices overcome these problems.

### Summary of the invention

Provided herein is a device (100) for placement of one or more skin-contacting electrodes on a subject (50) comprising:
- a planar support (120) having:
- a longitudinal main body portion (140) having a posterior end (10), an anterior end (12), an inferior side (14) and a superior side (16);
- a side arm portion (170) having a near end (18), a far end (20), an inferior side (14) and a superior side (16), wherein the side arm portion is joined at its near end (18) to the main body portion (140) towards the posterior end (10) of the main body portion (140);
- wherein a portion of the main body portion (140) contains a landing region (142, 142') for mechanical and electrical dismountable attachment of an electronic unit (300) on the superior side (16);
- wherein the main body portion (140) is longitudinally flexible so as to be able to conform to a shape of the midline of the head (52) of the subject (50);
- wherein the side arm portion (170) is at least longitudinally flexible so as to be able to bend towards the anterior end (12) of the longitudinal main body portion (140);
- a plurality of signal-conducting elements (148a to d) that are skin-contacting electrodes or mounts for dismountable attachment of skin-contacting electrodes;
wherein the inferior side (14) of the main body portion (140) comprises at least one signal-conducting element (148a to c); and
wherein the inferior side (14) of the side arm portion (170) comprises at least one signal-conducting element (148d).

The support (120) may be configured for adoption of a native conformation that is planar, and an active conformation in which the main body portion (140) is curved longitudinally to conform to a shape of midline (54) of the head (52) of the subject (50).

The main body portion (140) may be configured to adopt the active conformation by application of tension force (T) to the main body portion (140) between the posterior end (10) and the anterior end (12).

The device (100) may be configured for attachment to a bodily-worn support (200), wherein the bodily-worn support (200) contains a supporting structure stably attachable to the subject's head, and configured for application of the tension force (T) to the main body portion (140).

The main body portion (140) may comprise a plurality of fixing elements (150) for dismountable attachment of the device (100) to the bodily-worn support (200).

The plurality of fixing elements (150) may be a combination of a plurality of posterior mounting apertures (152a, 152b, 152c) and an anterior mounting aperture (152d), configured to allow the application and adjustment of the tension force (T).

The landing region (142, 142') may comprise a plurality of electrical connectors (144a to d; 144a' to d') on the superior side (16) for dismountable electrical attachment of the electronic unit (300) to the landing region (142).

The landing region (142) may comprise at least one mechanical connector (146a to d) for dismountable mechanical attachment of the electronic unit (300) to the landing region (142), optionally wherein each electrical connector (144a to d; 144a' to d') is also a mechanical connector (146a to d) for dismountable mechanical attachment of the electronic unit to the landing region (142).

The main body portion (140) may be disposed with at least two discrete landing regions landing regions (142, 142'), one the landing region (142) positioned towards the posterior end (10) of the main body portion (140) and another landing region (142') positioned towards the anterior end (12) of the main body portion (140), and the electronic unit (300) is dismountably attachable to either (142, 142').

The at least one mechanical connector (146a to d) may be configured such that electronic unit (300) is dismountably attachable to the landing region (142, 142') in only one orientation.

At least a part of the planar support (120) may be non-stretchable, preferably, wherein the planar support (120) contains a non-stretchable region spanning at least the signal-conducting element(s) (148a to c) of the main body portion (140).

The planar support (120) may comprise:
- an outer inferior (14) layer that is outwardly exposed and skin-facing, wherein the main body portion (140) and the side arm portion (170) share a continuous outer inferior (14) layer,
- an outer superior (16) layer that is outwardly exposed and opposed to the outer inferior (14), wherein the main body portion (140) and the side arm portion (170) share a continuous outer superior (16) layer, and
- optionally at least one inner layer disposed between the outer superior (16) layer and the outer inferior (14) layer, wherein at least one inner layer is disposed with a plurality of electrical conductors for transmission of electrical signals between the plurality signal-conducting elements (148a to d) and the landing region (142, 142').

The device (100) may contain at least three signal-conducting elements (148a to c) for conduction of EEG signals, and optionally EEG, EMG, ECG, tDCS signals, disposed spatially separated in a longitudinal direction on the main body portion (140), positioned to coincide with the Fz, Pz, and Cz positions on the head (52) of the subject (50).

A distance between adjacent pairs of signal-conducting elements (148a-148b; 148b-148c) may be fixed, may be the same, and may be between 45 and 105 mm.

The side arm portion (170) may be configured for bending such that the signal-conducting element (148d) reaches the skin of the head (52) in a region posterior to the ear.

### Figure Legends

**FIG. 1** shows a plan view of a planar support as incorporated into the device presently provided.
**FIG. 2** shows directions in relation to the device that are superior, inferior, anterior, posterior, far end and near end.
**FIG. 3** shows a superior side of a main body portion of the present device and four electrical connectors.
**FIG. 4** shows an inferior side of a main body portion of the present device and three signal-conducting elements.
**FIG. 5A** shows a superior side of a side arm portion of the present device.
**FIG. 5B** shows an inferior side of a side arm portion of the present device and one signal-conducting element.
**FIG. 6** is similar to FIG. 1, showing two landing regions.
**FIG. 7** is similar to FIG. 1, showing fixing elements.
**FIG. 8** shows a wiring diagram of the presently provided device having one landing region.
**FIG. 9** shows a wiring diagram of the presently provided device having two landing regions.
**FIG. 10** is a view of the device presently provided mounted on the head of the subject.
**FIG. 11** is a view of the device presently provided mounted on the head of the subject, and held under tension by a bodily-worn support.
**FIG. 12** is similar to FIG. 10, wherein the headband is incorporated into a VR/AR headset.
**FIG. 13** is a frontal views of the head, indicating the midline.
**FIG. 14** is a schematic illustration of an electronic unit.
**FIG. 15** is a schematic illustration of an electronic unit attached to a device presently provided.

### Detailed description of invention

Before the present device of the invention is described, it is to be understood that this invention is not limited to particular device described, since such device may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention. The following detailed description, therefore, is not to be taken in a limiting sense, and the scope of the present invention is defined by the appended claims.

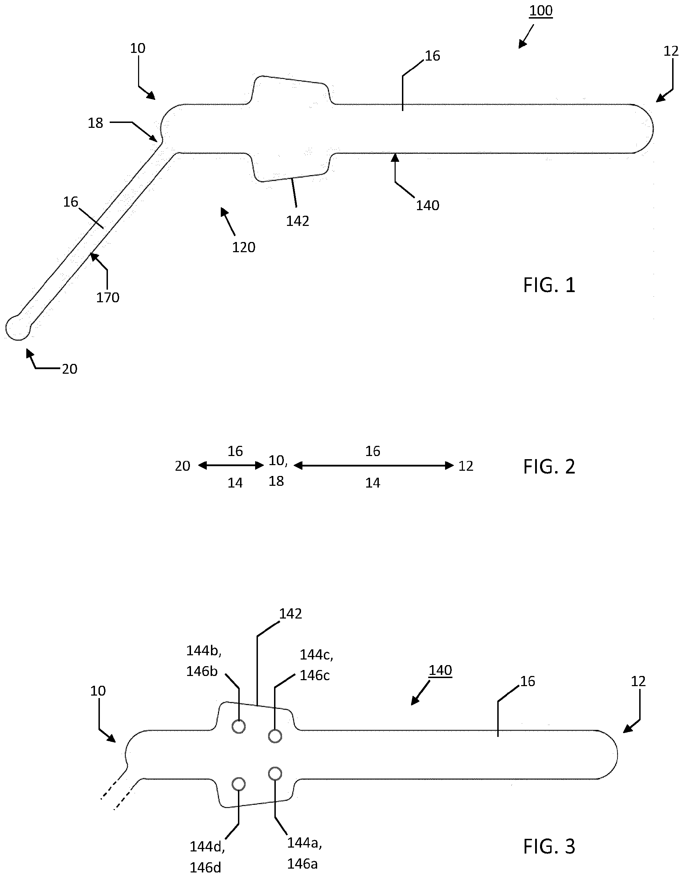

The terms superior, inferior, lateral, anterior, and posterior have been used here according to their ordinary meaning with respect to a body of a subject. The reference signs in **FIG. 1** and **FIG. 2** indicate the different directions used herein. The terms superior (16) and inferior (14) mean towards the head (vertex) and towards the feet respectively of an subject in particular when device (100) or main body portion (140) is mounted or to be on the subject's head. The terms anterior (10) and posterior (12) means towards the front and towards the back respectively of the subject in particular when device (100) or main body portion (140) is mounted or to be on the subject's head. The term near end (18) is used in relation to the side arm portion (170) and means the end of the side arm portion (170) towards the main body portion (140). The term far end (20) is used in relation to the side arm portion (170) and means the end of the side arm portion (170) away the main body portion (140). The term lateral means towards the left and/or right side of the subject.

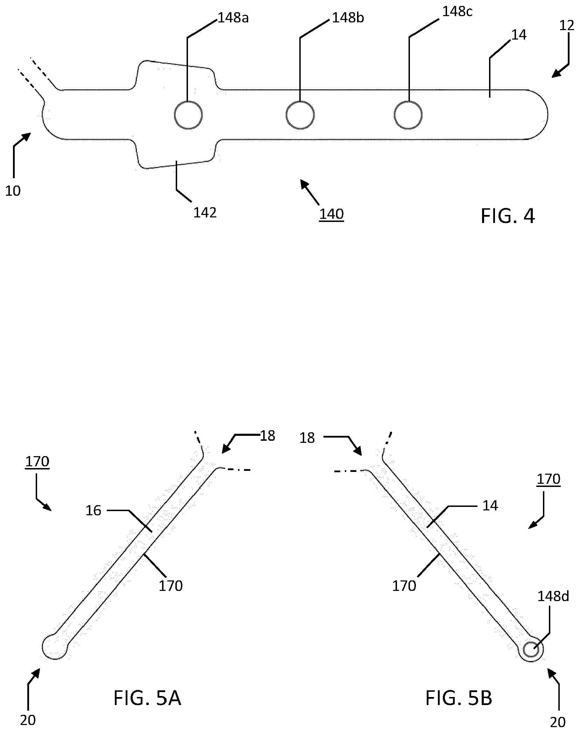

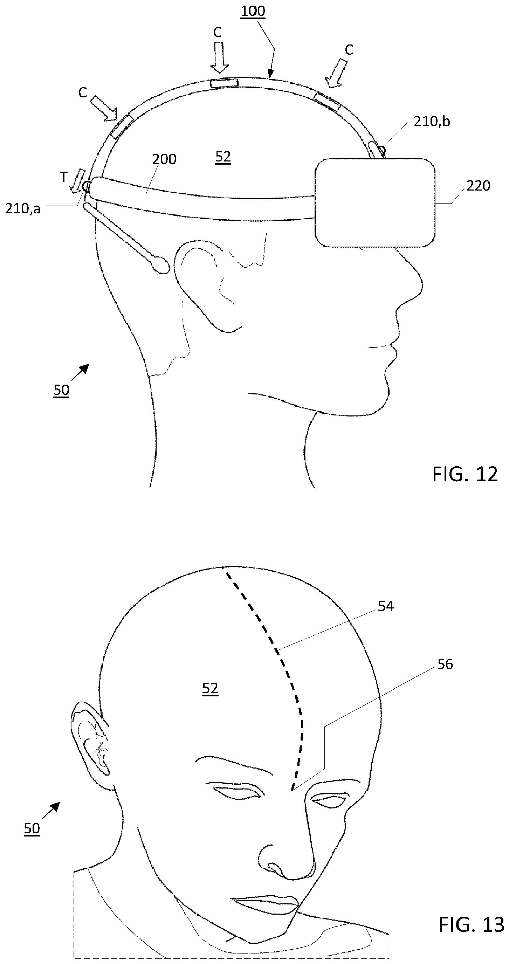

Provided herein is a device (100) for placement of one or more skin-contacting electrodes on a subject. Exemplary devices (100) are shown in various views in **FIGs. 1** and **3** to **12, 15.** The device (100) comprises a flexible planar support (120) and a plurality of signal-conducting elements (148a to d) that are skin-contacting electrodes or mounts for dismountable attachment of skin-contacting electrodes. One or more of the skin-contacting electrodes may be configured for detection of electrical signals generated by the body (e.g. EEG and/or EMG and/or ECG signals), more in particular neural signals, more in particular electrical signals generated by the brain (*e.g*. EEG signals). One or more of the skin-contacting electrodes may be configured for conduction of (stimulatory) electrical signals to the body, more in particular to a neural system, more in particular to the brain (*e.g*. in tDCS). The device (100) is configured for attachment to a bodily-worn support (200) such as a headband, head garment (cap, bonnet) or AR (augmented reality) / VR (virtual reality) headset so that the support (120) is under longitudinal tension and applies a pressure so the skin-contacting electrodes to achieve an optimum orientation and compressing force against the skin of the subject's head (52), in particular along at least a part of the midline of the head (52). **FIG. 13** shows an illustration of a midline (54) of a subject's (50) head (52), which extends from the nasion (56). The inventors have found that having a flexible planar support (120) provides an infinite conformity to the shape of the head (52) of the subject (50) that provides comfort to the wearer and better electrode-skin contact. Thus conformity to head shape and electrode positioning are simple and time effective to carry out. Other advantages described elsewhere herein include environmental friendliness, cost reduction for manufacture, ease of placement, single use/multi use options, strong signal detection.

A signal-conducting element (148a to d) may be a skin-contacting electrode; in other words, the skin-contacting electrode may be non-removably attached to (*e.g*. embedded in) the planar support (120). A signal-conducting element (148a to d) may be a mount for dismountable attachment of a skin-contacting electrode; in other words, the skin-contacting electrode may dismountably attach to the signal-conducting element (148a to d). Dismountable attachment may be realised through different mechanisms such as a press-stud, conductive contact adhesive.

One or more of the skin-contacting electrodes (148a to d) may be configured for detection of electrical signals generated by the body (*e.g*. EEG, EMG, ECG signals), more in particular neural signals, more in particular electrical signals generated by the brain (*e.g*. EEG signals). One or more of the skin-contacting electrodes (148a to d) may be configured for conduction of (stimulatory) electrical signals to the body, more in particular to a neural system, more in particular to the brain (*e.g*. tDCS). A single skin-contacting electrode may have a capacity for detection of signals from the body and/or transmission of stimulatory signals to the body. A single skin-contacting electrode may have a capacity to collect one or more of EEG, EMG, ECG signals, and/or to transmit stimulatory tDCS signals. The processing of the bodily signals may extract the relevant EEG, EMG, ECG information from the single skin-contacting electrode.

The skin-contacting electrode may have a flat surface. The skin-contacting electrode have a surface containing a plurality of conductive protrusions for passage through hair of the subject (50), and for contact with the skin. The skin-contacting electrode may be or may not be adhesive. The skin-contacting electrode may be a dry-contact electrode. The skin-contacting electrode may be pre-wetted. The skin-contacting electrode may be pre-gelled. The skin-contacting electrodes may be hard or soft. The skin-contacting electrode may be a wet-contact electrode.

The planar support (120) comprises a longitudinal main body portion (140) having a posterior end (10), an anterior end (12), an inferior side (14) and a superior side (16). The main body portion (140) is longitudinally flexible so as to be able to conform to a shape of the midline (54) of the head (52) of the subject (50). A portion of the main body portion (140) contains a (discrete) landing region (142) for mechanical and electrical dismountable attachment of an electronic unit on the superior side (16).

The planar support (120) further comprises a side arm portion (170) having a near end (18), a far end (20), an inferior side (14) and a superior side (16). The side arm portion is joined at its near end (18) to the main body portion (140) towards the posterior end (10) of the main body portion (140). The side arm portion (170) is at least longitudinally flexible so as to be able to bend towards the anterior end (12) of the longitudinal main body portion (140) (such that the far end (20) can contact the skin of the head (50) in a region posterior to the ear).

At least a part of the planar support (120) may be non-stretchable. At least a part of the planar support (120) may be non-stretchable in a longitudinal direction. The planar support (120) may be entirely non-stretchable. Non-stretchable means that there is no significant change in dimension in a direction in which a tensile force is applied. A non-stretchable region may span at least the signal conducting elements of the main body portion (140). A stretchable region may be at or towards the posterior (10) end of the main body portion (140), and /or at or towards the anterior (12) end of the main body portion (140). The planar support (120) may have one or more regions that are non-stretchable, and one or more regions that are stretchable. The posterior end may be stretchable so that additional tension is applied to the head of the subject.

The support (120) is configured to be able to adopt a native conformation that is planar, and an active conformation in which the main body portion (140) is curved longitudinally to comply with a curvature of the midline (54) of the head (52) of the subject (50). The native and active conformations are preferably not stable inherently. The active conformation is stabilisable with the application of a tension force between the posterior end (10) and the anterior end (12) of the main body portion (140) while on the head (52) of the subject (50). The active conformation stabilisable with the bodily-worn support. The native conformation is typically stabilised by accompanying packaging that biases the support (120) in the planar conformation.

The support (120), in particular the longitudinal main body portion (140), is configured to be placed under tension in a longitudinal direction, thereby bringing the skin-contacting electrodes into electrical contact with the skin of the subject (50). The inventors have found that by applying a tensile force to the longitudinal main body portion (140), the signal quality from the skin-contacting electrodes is improved; the tensile force not only increases pressure against the skin per electrode in an inferior direction, but also induces rotation on each skin-contacting electrode relative to the longitudinal main body portion (140) which brings it into a new direction conforming with the curvature of the head (52). Thus each skin-contacting electrode surface achieves a maximal contact area, requiring only application of tension, and avoids the need to individually adjust the orientation of each skin-contacting electrode. Accordingly, the device can be placed quickly on the head (52) of the subject (50), and with a minimal intervention from the user. In addition, the flexibility of the planar support affords more comfort to the wearer.

The main body portion (140) and the side arm portion (170) may be formed from a continuous sheet of support (120).

The planar support (120) may be formed from 1, 2 or 3 or more layers. A layer contains a sheet of material such as paper, cardboard, fabric, non-woven, polymeric. A layer may be coated, printed on, or otherwise modified. In a multi-layer support (120), the layers are typically joined together using adhesive and/or heat bonding.

The support (120) may comprise an outer superior (16) layer that is outwardly exposed. It is opposed to a skin-facing side of the support. The outer superior (16) layer is flexible at least in a longitudinal direction.

At least a part of the outer superior (16) layer is preferably non-stretchable at least in a longitudinal direction. At least a part of the outer superior (16) layer may be non-stretchable in a longitudinal direction. The outer superior (16) layer may be entirely non-stretchable. A non-stretchable region may span at least the signal conducting elements of the outer inferior (14) in the main body portion (140); thereby distances between pairs of signal-conducting elements (148a to c) is constant. A stretchable region may be at or towards the posterior (10) end of the outer inferior (14) in the main body portion (140), and /or at or towards the anterior (12) end of the outer inferior (14) in the main body portion (140). The outer superior (16) layer may have one or more regions that are non-stretchable, and one or more regions that are stretchable.

An outward facing surface of the outer superior (16) layer may contain one or more visual markings. Examples of visual markings include demarcation of the landing region(s), indication of the anterior and/or posterior direction, indication of size, indication of adjustment position, a measurement gauge, identification tag (see elsewhere herein). The visual markings may be printed, embossed, engraved, cut out, etc.

The main body portion (140) and the side arm portion (170) may share a continuous outer superior (16) layer. The main body portion (140) and the side arm portion (170) may be formed from a continuous sheet of outer superior (16) layer. The outer superior (16) layer may be made from any material that is inherently flexible or that is processed to have flexibility (e.g. with flexibility slots or cuts) Examples include, polymers such as thermoplastic, polyurethane, PET, polypropylene, plastic film, fabrics, paper, cardboard, or any other material that has appropriate mechanical properties.

The support (120) may comprise an outer inferior (14) layer that is inwardly exposed. It is on the skin-facing side of the support. The outer inferior (14) layer is flexible at least in a longitudinal direction. At least a part of the outer inferior (14) layer is preferably non-stretchable at least in a longitudinal direction.

At least a part of the outer inferior (14) layer may be non-stretchable in a longitudinal direction. The outer inferior (14) layer may be entirely non-stretchable. A non-stretchable region may span at least the signal conducting elements of the outer inferior (14) in the main body portion (140); thereby distances between pairs of signal-conducting elements (148a to c) is constant. A stretchable region may be at or towards the posterior (10) end of the outer inferior (14) in the main body portion (140), and /or at or towards the anterior (12) end of the outer inferior (14) in the main body portion (140). The outer inferior (14) layer may have one or more regions that are non-stretchable, and one or more regions that are stretchable.

An outward facing surface of the outer inferior (14) layer may contain one or more visual markings. Examples of visual markings include demarcation of the landing region(s), indication of the anterior and/or posterior direction, indication of size, indication of adjustment position, a measurement gauge, identification tag (see elsewhere herein). The visual markings may be printed, embossed, engraved, cut out, etc.

The main body portion (140) and the side arm portion (170) may share a continuous outer inferior (14) layer. The main body portion (140) and the side arm portion (170) may be formed from a continuous sheet of outer inferior (14) layer. The outer inferior (14) layer may be made from any material that is inherently flexible or that is processed to have flexibility (*e.g*. with flexibility slots or cuts). Examples include, polymers such as thermoplastic; polyurethane, PET, polypropylene, plastic film, fabrics or any other material that has appropriate mechanical properties.

The support (120) may comprise at least one inner layer disposed between the outer superior (16) layer and the outer inferior (14) layer. The at least one inner layer is flexible at least in a longitudinal direction. At least a part of the at least one inner layer is preferably non-stretchable at least in a longitudinal direction. At least a part of the at least one inner layer may be non-stretchable in a longitudinal direction. The at least one inner layer may be entirely non-stretchable. The at least one inner layer may be non-stretchable at least in the regions between the plurality of signal-conducting elements (148a to c); thereby distances between pairs of signal-conducting elements (148a to c) is constant. The main body portion (140) and the side arm portion (170) may share a continuous inner layer. The main body portion (140) and the side arm portion (170) may be formed from a continuous sheet of inner layer. The at least one inner layer may be made from any material that is inherently flexible or that is processed to have flexibility (*e.*g. with flexibility slots or cuts). Examples include, polymers such as thermoplastic; polyurethane, PET, polypropylene, plastic film, fabrics or any other material that has appropriate mechanical properties.

The at least one inner layer may be a layer disposed with a plurality of conductors (156; 156,a; 156,b; 156,c; 156,d; 156,a'; 156,b'; 156,c'; 156,d') (*e.g*. printed conductors, wire conductors) for transmission of electrical signals between the plurality signal-conducting elements (148a to d) and the landing region (142, 142'), in particular the plurality of electrical connectors (144a to d; 144a to d). The plurality of electrical conductors may be, for example, printed on, or sandwiched within at least one inner layer. The conducting material may be any such as silver, gold, copper, or carbon inks.

The support (120) may comprise a combination of the outer superior (16), outer inferior layer (14), and the one or more inner layers.

The support (120) may comprise a combination of the outer superior (16) and outer inferior layer (14). Components such as the plurality of conductors, RFID tag, may be disposed sandwiched between the outer superior (16) and outer inferior layer (14).

The support (120) may contain only one layer: it may be the outer superior (16) or the outer inferior layer (14). Components such as the signal-conducting elements (148a to d), electrical connectors (144a to d), mechanical connector, may be attached to the one layer. Components such as the plurality of conductors, RFID tag, may be disposed on a surface of the one layer, attached by bonding (*e.g*. adhesive or heat). Electrical insulation where needed may be provided by a coating (*e.g*. varnish) or protective patch.

The layers may be joined together by any process or means, *e.g*. adhesive, heat bonding, laminating. The adhesive may be disposed between the layers and/or at the edges of the layers. The layers may be joined together to prevent ingress of moisture or liquid between the outer superior (16) layer and outer inferior (14) layer. A planar support is economical to store and transport. A layered planar support is easy to manufacture and assemble: the respective layers can be automatically cut (*e.g*. on an xy cutting machine, or from a shaped cutter) from single layers, and a major assembly step is an alignment of layers which can be performed automatically because of their planar form. The use of a multi-layer planar support is that electronic parts can be sealed within the outer superior (16) layer and outer inferior (14) layer, thereby making the device easier to clean with steam or liquid in cases where it is reused. The device has only two major surfaces, and is devoid of complex parts, meaning that cleaning and drying are thorough and fast.

The main body portion (140) of the planar support (120) is flexible and longitudinal. Different views of the main body portion (140) are shown in **FIGs. 1, 2****,** **4****,** **6** to **9.** It may have a strap-like appearance. The main body portion (140) may have a maximum width (in a non-landing region) of 8 to 12 cm. The main body portion (140) may have a minimum width of 1 cm. The main body portion (140) may have a maximum length of 40 to 60 cm. The main body portion (140) may have a minimum length of 15 cm.

The main body portion (140) is configured for conformity to a shape of midline (54) of the head (52) of the subject (50) by application of tension force to the main body portion (140) between the posterior end (10) and the anterior end (12). In particular the tension force may applied between the posterior end (10) and the anterior end (12) of the main body portion (140) in an inferior direction.

At least a part of the main body portion (140) may be non-stretchable. At least a part of the main body portion (140) may be non-stretchable in a longitudinal direction. The main body portion (140) may be entirely non-stretchable. The main body portion (140) may be non-stretchable at least in a region spanning the plurality of signal-conducting elements (148a to c); thereby distances between pairs of signal-conducting elements (148a to c) is constant. The main body portion (140) may be non-stretchable at least in a region spanning the anterior end (12) to the posterior (10)-most signal-conducting element (148a); thereby distances between pairs of signal-conducting elements (148a to c) is constant. The non-stretchable quality provides for reproducible placement of the signal-conducting elements (148a to c) with respect to the midline (54) of the head (52). The positions of the signal-conducting elements (148a to c) can be fixed during manufacture (e.g. one size fits all (adult); one size fits all (child)), and they will not change during placement because of the non-stretchable quality. The user (e.g. health practitioner, subject, carer) placing one end of the device with reference to a bodily feature (e.g. the nasion) will bring the signal-conducting elements (148a to c) into alignment with the relevant regions of the midline (54) of the head (52) of the subject (50), without a need for further adjustment. Thus electrode positioning is simple and time effective to carry out.

The main body portion (140) is configured to be able to adopt a native conformation that is planar, and an active conformation in which the main body portion (140) is curved longitudinally to comply with a curvature of the midline (54) of the head (52) of the subject (50). The native and active conformations are not stable inherently. The active conformation stabilisable with the application of a tension force between the posterior end (10) and the anterior end (12) while on the head (52) of the subject (50). The active conformation stabilisable with the bodily-worn support.

A portion of the planar support (120), in particular, a portion of the main body portion (140) is disposed with one or more landing regions (142,142') for mechanical and electrical dismountable attachment of an electronic unit on the superior side (16). Each landing region is discrete. The landing region (142,142') may be discerned by an increase or decrease in the width of the main body portion (140). The portion of the main body portion (140) in the landing region (142,142') may be wider than main body portion (140) in a non-landing region. The landing region (142,142') may be additionally or alternatively be discerned by a marking on the superior side (16) of the main body portion (140).

The landing region (142 or 142') comprises a plurality of electrical connectors (144a to d) on the superior side (16) for dismountable electrical attachment of the electronic unit to the landing region (142).

The landing region (142 or 142') may comprise at least one mechanical connector (e.g. press-stud, Velcro, magnet) (146a to d) for dismountable mechanical attachment of the electronic unit to the landing region (142 or 142'). Some or all of the electrical connectors (144a to d) may each also function a mechanical connector (e.g. press-stud) (146a to d) for dismountable mechanical attachment of the electronic unit to the landing region (142 or 142').

The electrical connectors (144a to d) may be each spatially separated from each other.

The electrical connectors (144a to d) may be each spatially separated from the at least one mechanical connector (*e.g*. press-stud, Velcro, magnet) (146a to d).

The electrical connectors (144a to d) may be each attached (only) to the outer superior (16) layer, and optionally to one or more inner layers of the support (120).

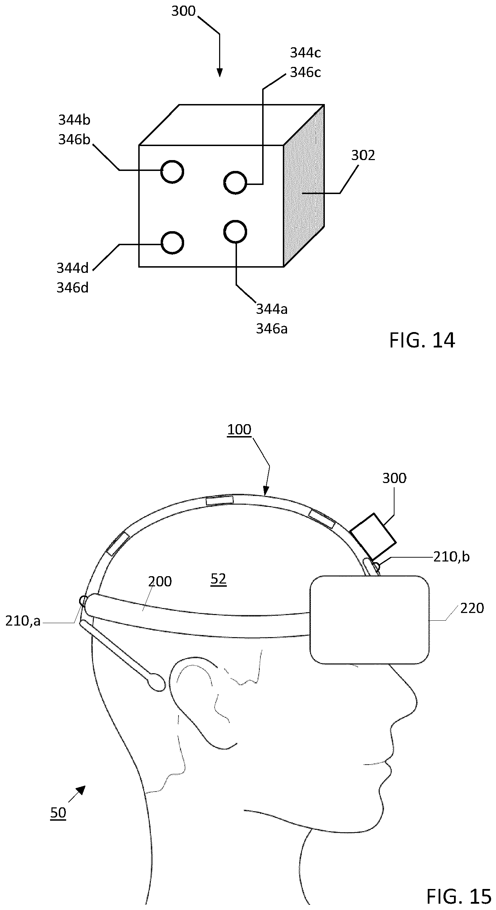

The at least one mechanical connector (*e.g*. press-stud, Velcro, magnet) (146a to d) may be configured for dismountable mechanical attachment of the electronic unit to the landing region (142) in only one orientation. This may be achieved by a geometry of the at least one mechanical connector which permits dismountable mechanical attachment of the electronic unit in only one orientation. The geometry may have a rotational asymmetry *i.e.* the mechanical connector looks the same only after a complete rotation around an axis of rotation. As shown in **FIGs. 3****,** **8** and **9****,** the geometry of the four mechanical connectors (146a to d) permit mechanical attachment of the electronic unit (*e.g*. **FIG. 14**) in only one orientation. According to one example, there may be four mechanical connectors arranged in a trapezoid geometry, obliging placement of the electronic unit in only one direction. An advantage of attachment of the electronic unit in only on orientation is that it ensures a correct electrical connection between the electronic unit and the support; it becomes impossible to electrically connect both in the wrong orientation because the electronic unit would not be able to attach to the support when improperly placed.

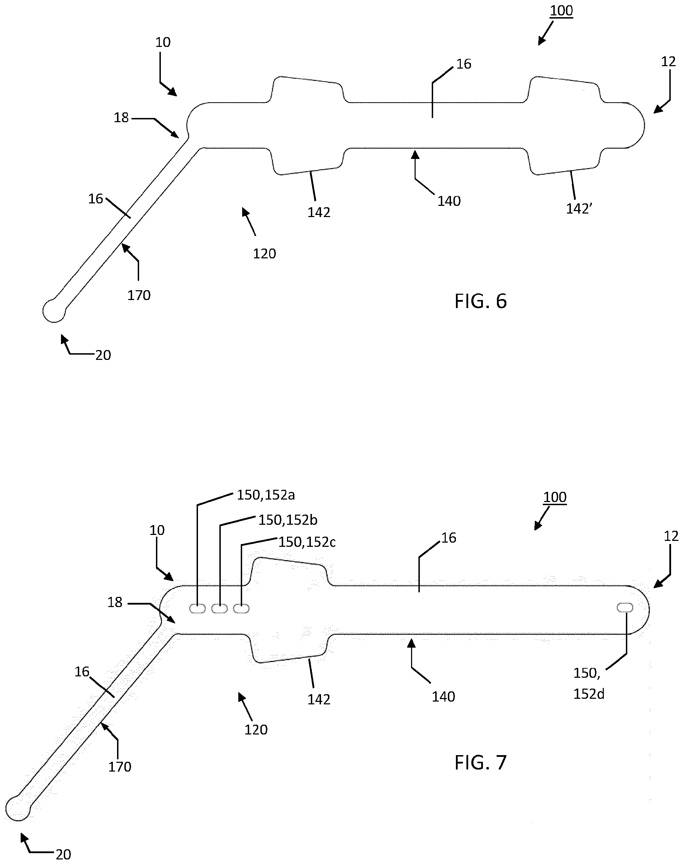

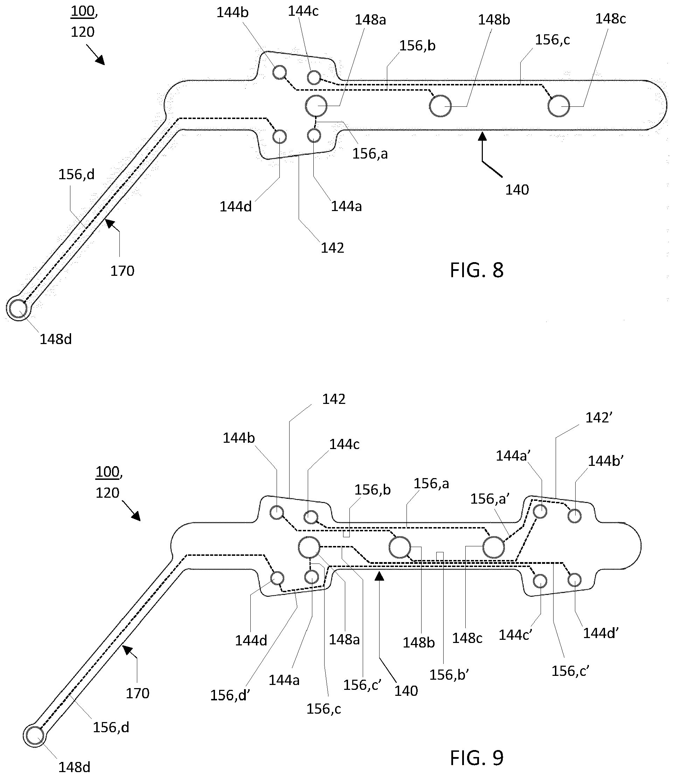

Where the device (100) is disposed with at least two discrete landing regions landing regions (142, 142'), one the landing region (142) may be positioned towards the posterior end (10) of the main body portion (140) and another landing region (142') may be positioned towards the anterior end (12) of the main body portion (140), and the electronic unit is dismountably attachable to either (142, 142'). **FIGs. 6** and **9****,** shown an example of the device with two landing regions. The geometry of the plurality of electrical connectors (144a to d; 144a' to d') and of the at least one mechanical connector may be the same in both landing regions (142, 142'). In both landing regions (142, 142') each electrical connector at the same corresponding position (144a-144a', 144b-144b', 144c-144c', 144d-144d') is electrically connected to the same signal-conducting element (148a to d). An advantage of having the same geometry in different landing regions is that allows a choice of attachment by the same electronic unit to more than one position on the support, depending, for instance, on placement of the subject (50) (*e.g*. prostrate or supine).

The device (100), in particular the planar support (120) is configured for attachment to a bodily-worn support (200). The attachment may be achieved by any mechanism (*e.g*. standard mechanism, clamps, hooks, Velcro). The attachment to the bodily-worn support (200) may be direct; in other words, the device (100) is in contact with the bodily-worn support (200). The attachment to the bodily-worn support (200) may be indirect; the device (100) attaches to the bodily-worn support (200) *via* one or more intervening elements (*e.g.* a bridging strap, elasticated or non-elasticated). Attachment to the bodily-worn support (200) allows application of tension (T) to the planar support (120), which in turn applies a compressive force (C) to the head (52). The compressive force (C) contributes to a strong electrical contact between the skin and electrode. The attachment allows the main body portion (140) to adopt an active conformation.

The bodily-worn support (200) is any type of mounting that allows tension to be applied to the main body portion (140). The bodily-worn support (200) typically contains a supporting structure stably attachable to the subject's head. The bodily-worn support (200) typically comprises a ring-shaped support, such as an elastic or manually-adjustable or automatically-adjustable band, that applies a compressive force to the subject's head (52) to stabilise its position. The bodily-worn support (200) may be incorporated into a headband, or a head garment (cap, bonnet), or a VR or AR headset having a VR or AR visor (220).

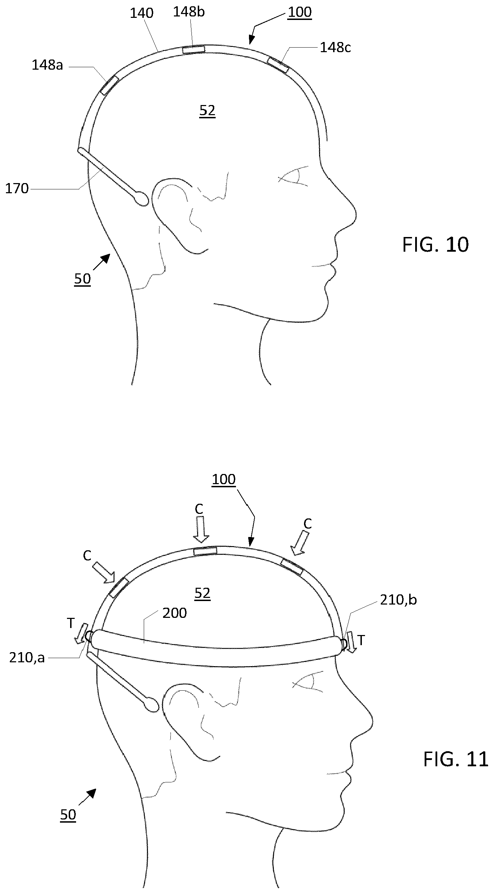

The device (100), in particular the support (120), may further comprise one or more (e.g. a plurality) fixing elements (150) for dismountable attachment of the device attachment to the bodily-worn support (200). A device disposed with one or more fixing elements (150) is shown in **FIG. 7****.** Exemplary bodily-worn supports (200) are shown in **FIG. 11** (headband without VR or AR headset) and in **FIG. 12** (headband incorporated in VR or AR headset).

The bodily-worn support (200) may be disposed with one or more complementary fixing elements (210,a; 210,b) that engage with the device (100) fixing elements (150) or with the one or more intervening elements, thereby allowing attachment of the device (100), in particular of the support (120). The bodily-worn support (200) provides a stable supporting structure, allowing the support (120) to be placed under tension (T) in a longitudinal direction, thereby bringing the skin-contacting electrodes into electrical contact with the skin of the subject (50).

The at least one fixing element (150) may be a mounting aperture (152a, 152b, 152c, 152d) that is an aperture (window or hole) in the support (120) configured for attachment of the device (100) to the bodily-worn support (200). The mounting aperture 152a, 152b, 152c, 152d) may be configured for engagement with a complementary fixing element (210,a; 210,b) of the bodily-worn support (200) or of the one or more intervening elements that incorporates a hook. The mounting aperture (152a, 152b, 152c, 152d) may be reinforced (e.g. with a peripheral lining). The mounting aperture (152a, 152b, 152c, 152d) may have a circular, rectangular, round-edged rectangular, square, oval, or triangular footprint.

The device (100), in particular the main body portion (140) of the support (120), may comprise at least one fixing element (150) that is a posterior mounting aperture (152a, 152b, 152c) disposed towards the posterior end (10) of the main body portion (140).

The device (100), in particular the main body portion (140) of the support (120), may comprise a plurality of fixing elements (150) that are a plurality of posterior mounting apertures (152a, 152b, 152c) disposed towards the posterior end (10) of the main body portion (140), wherein each posterior mounting aperture (152a, 152b, 152c) is spatially separated along a longitudinal direction of the main body portion (140). The spatial separation allows for an adjustment of tension applied by the main body portion (140)) based on the head size of the subject. A visual marking may be placed adjacent to each aperture (e.g. L, M, S) indicating a size created by selection of that aperture, ensuring optimum electrode location.

The device (100), in particular the main body portion (140) of the support (120), may comprise at least one fixing element (150) that is an anterior mounting aperture (152d) disposed towards the anterior end (12) of the main body portion (140).

The plurality of fixing elements (150) that are a combination of a plurality of posterior mounting apertures (152a, 152b, 152c) and an anterior mounting aperture (152d) allow an application and adjustment of tension (T) applied by the main body portion (140).

The combination of the plurality of posterior mounting apertures (152a, 152b, 152c) and the anterior mounting aperture (152d) allow an application and adjustment of tension applied by the main body portion (140). While the main body portion (140) has an infinitely adjustable conformation to conform quickly to any head shape, the mounting apertures (152a, 152b, 152c, 152d) allow for a variation in head size which have been standardised into major groups (*e.g*. S, M, L). By using apertures, manufacture of the device is simplified: steps for placement additional mechanisms are avoided, allowing a reduction in costs, and suitability for one-time use/limited reuse. Further, the manufacturing process can be readily modified for placement of more, less of differently placed apertures, depending, for instance, on differences between population sub-groups or regions. Placement of a plurality mounting apertures (152a, 152b, 152c) at a posterior end of the main body portion (140) prevents that a trailing end of the main body portion (140), created when the innermost posterior and anterior apertures are selected, contacts the eyes.

The main body portion (140) may comprise at least two signal-conducting elements (148a to c) for conduction of signals disposed spatially separated in a longitudinal direction on the main body portion (140). One or more of the skin-contacting electrodes (148a to c) on the main body portion (140) may be configured for detection of electrical signals generated by the body (*e.g*. EEG, EMG, ECG signals), and/or for conduction of (stimulatory) electrical signals to the body (*e.g*. tDCS).

The main body portion (140) may comprise at least three signal-conducting elements (148a to c) for conduction of EEG signals (optionally additionally for conduction of EMG, ECG and tDCS signals) disposed spatially separated in a longitudinal direction on the main body portion (140). A distance between adjacent pairs of signal-conducting elements (148a-148b; 148b-148c) may be fixed (non-adjustable). A distance between adjacent pairs of signal-conducting elements on the main body portion (140) may be between 45 and 105 mm. A distance between adjacent pairs of signal-conducting on the main body portion (140) elements may be the same. The at least three signal-conducting elements (148a to c) for conduction of signals may be positioned to coincide with the Fz, Pz, and Cz positions as defined by the 10-20 system for EEG placement.

By fixing the distances of the signal-conducting elements (148a to c), the user can more quickly attach the device to the subject, as the possibility for adjustments is redundant. Because there is no adjustment, the possibility for errors is reduced. The faster application reduces time spent per patient, thereby reducing treatment costs and strain on resources.

A distance between adjacent pairs of signal-conducting elements (148a-148b; 148b-148c) may be the same and be between 45 and 105 mm, and a distance from the anterior-most signal-conducting element (148c) to the (anterior edge) of the anterior mounting aperture may be between 40 and 80 mm.

The signal-conducting elements (148a to c) may be each attached (only) to the outer inferior (14) layer, and optionally to one or more inner layers of the support (120).

The side arm portion (170) may be longitudinal. The side arm portion (170) in a native (planar) conformation of the support (120) may project from a (left and/or right) lateral side of the main body portion (140). The side arm portion (170) in a native (planar) conformation of the support (120) may project at an obtuse angle from a (central) longitudinal axis of the main body portion (140). The signal-conducting element (148d) on the inferior side (14) of the side arm portion (170) may be an adhesive skin-contacting electrode. A maximum width of the side arm portion (170) may be less than a maximum width of the main body portion (140). A maximum length of the side arm portion (170) may be less than a maximum length of the main body portion (140). The side arm portion (170) may have a maximum length of 18-22 cm. The side arm portion (170) may be configured (in the active conformation) to bend such that the signal-conducting element (148d) can reach the skin of the head (52) in a region posterior to the ear. At least a part of the side arm portion (170) may be non-stretchable. At least a part of the side arm portion (170) may non-stretchable in a longitudinal direction. The side arm portion (170) may be entirely non-stretchable.

The side-arm portion (170), may comprise at least one (preferably only one) signal-conducting element (148d) for conduction of signals.

The signal-conducting element (148d) on the side-arm portion (170) may be a skin-contacting electrode; in other words, the skin-contacting electrode may be embedded in the support. The signal-conducting element (148d) on the side-arm portion (170) may be a mount for dismountable skin-contacting ground electrode; in other words, the skin-contacting electrode may dismountably attach to the signal-conducting element (148d). This skin-contacting electrode may be a ground electrode (*e.g*. for EEG, EMG, tDCS, ECG signals). This skin-contacting electrode may be for collection ECG signals.

Placing the signal-conducting element (148d) for the ground electrode on a side-arm portion (170) separated from other signal-conducting elements (148a-c) (*e.g*. EEG EMG, tDCS, ECG signals) reduces noise interference: the side arm portion (170) compels the user to position the signal-conducting element (148d) far away from the head midline (54). Moreover, integration of the ground electrode into a planar support (120) reduces manufacturing costs: the side arm portion (170) and main body portion may be manufactured from the same planar support *e.g*. by cutting out from sheets of material, thereby avoiding additional assembly steps. The reduction in costs increases viability for a one-time use/limited reuse device.

The plurality of electrical connectors (144a to d) are electrically connected to the plurality of signal-conducting elements (148a to d). The electrical connections within the device may be realised by a plurality of electrical conductors (156; 156,a; 156,b; 156,c; 156,d; 156,a'; 156,b'; 156,c'; 156,d') for transmission of electrical signals. Each electrical connector (144a to d; 144a' to d') may be electrically connected to no more than one, preferably only one, signal-conducting element (148a to d). Examples of wiring diagrams are shown in **FIGs. 8** and **9****.**

Where there is one landing region (142) (*e.g*. **FIG. 8**) (only) one electrical connector (144a to d) of the landing region (142) and one signal-conducting element (148a to d) may be mutually electrically connected. In **FIG. 8****,** electrical connectors (144a to d) are connected to signal-conducting element (148a to d) by electrical conductors (156,a to d) respectively.

Where there are two or more landing regions (142, 142') (only) one electrical connector (144a to d) of the landing region (142), and (only) one electrical connector (144a' to d') of the further landing region (142'), and (only) one signal-conducting element (148a to d) are mutually electrically connected. The position of the electrically mutually connected electrical connectors (144a-144a'; 144b-144b'; 144c-144c'; 144d-144d') is the same in each landing region (142, 142'). In **FIG. 9****,** electrical connectors (144a to d) of one landing region (142) are connected to signal-conducting element (148a to d) by electrical conductors (156,a to d) respectively, and electrical connectors (144a' to d') of the other landing region (142') are connected to signal-conducting element (148a to d) by electrical conductors (156,a' to d') respectively.

The electrical connections within the device may be realised by one or more inner layers of the support (120) containing a plurality of electrical conductors (156; 156,a; 156,b; 156,c; 156,d; 156,a'; 156,b'; 156,c'; 156,d') for transmission of electrical signals. At least one inner layer may be disposed with the plurality of electrical conductors (156; 156,a; 156,b; 156,c; 156,d; 156,a'; 156,b'; 156,c'; 156,d'). The plurality of electrical conductors may be, for example, printed on, or sandwiched within at least one inner layer. The conducting material may be any such as silver, gold, copper, or carbon inks. By using least one inner layer disposed with the plurality of electrical conductors, manufacturing is simplified, thereby reducing costs; once the conducting inner layer is aligned with the outer superior (16) layer and outer inferior (14) layer, the electrical conductors will be correctly aligned for connection to the electrical connectors (144a to d) and the signal conducing elements (148a to d) and assembly can be achieved by a pressing together of planar parts e.g. in a flat-bed press. Because the support is planar, complex three dimensional assembly methods and equipment are avoided. The reduction in manufacturing costs allow the device to have a limited life as the cost of replacement is low.

The electrical connectors (144a to d; 144a' to d') may be each attached to the outer superior (16) layer and to one or more inner layers of the support (120) containing a plurality of electrical conductors (156; 156,a; 156,b; 156,c; 156,d; 156,a'; 156,b'; 156,c'; 156,d'). The signal-conducting elements (148a-d) may be each attached to the outer inferior (14) layer and to one or more inner layers of the support (120) containing a plurality of conductors. Alternatively, or in addition, an electrical connection between two points may be achieved by a conventional electrical wire.

As mentioned elsewhere herein, an electronic unit (300) can mechanically and electrically dismountably attach to the superior side (16) of the main body portion (140), in particular, to a landing region (142,142') of the main body portion (140). A non-limiting schematic illustration of an electronic unit (300) is shown in **FIG. 14****.** The electronic unit (300) is configured for conversion of the bodily detected signals (*e.g*. EEG, EMG, ECG signals) into digital information for storage and/or display and/or wireless or wired transmission. Alternatively or in addition, the electronic unit (300) may be configured for generation of stimulatory signals (*e.g.* tDCS).

The electronic unit (300) comprises a plurality of complementary electrical connectors (344a to d) that electrically connect with the plurality of electrical connectors (144a to d) on main body portion (140). The electronic unit (300) may further comprise at least one complementary mechanical connector (*e.g*. press-stud, Velcro, magnet) (346a to d) for dismountable mechanical attachment of the electronic unit to the landing region (142 or 142'). Some or all of the complementary electrical connectors (344a to d) may each also function a complementary mechanical connector (*e.g*. press-stud) (346a to d) for dismountable mechanical attachment of the electronic unit (300) to the landing region (142 or 142'). The complementary electrical connectors (344a to d) may be each spatially separated from each other. The complementary electrical connectors (344a to d) may be each spatially separated from the at least one mechanical connector (*e.g*. press-stud, Velcro, magnet) (346a to d). The at least one complementary mechanical connector (*e.g*. press-stud, Velcro, magnet) (346a to d) may be configured for dismountable mechanical attachment of the electronic unit to the landing region (142) in only one orientation. This may be achieved by a geometry of the at least one complementary mechanical connector which permits dismountable mechanical attachment of the electronic unit in only one orientation. The geometry may have a rotational asymmetry *i.e.* the complementary mechanical connector looks the same only after a complete rotation. As shown in **FIG. 14** the geometry of the four mechanical connectors (146a to d) permit mechanical attachment of the electronic unit in only one orientation.

The electronic unit (300) typically comprises a housing (302). The complementary electrical connectors (344a to d) and optional at least one complementary mechanical connector (e.g. press-stud, Velcro, magnet) may be disposed on an exterior of the housing. The housing exterior may be washable. The housing exterior may be wrappable in a protective cover e.g. to maintain a hygiene environment during a measurement. The housing protects components within.

The electronic unit (300) comprises one or more electrical components for the collection of signals (*e.g.* EEG, EMG, ECG), and/or for the generation of stimulatory signals *(e.g.* tDCS). Examples of components include one or more of a signal amplifier(s), analogue to digital converter, digital to analogue converter, storage device for storage of the signal data, communication interface (*e.g.* wireless (*e.g.* Bluetooth, WiFi, Zigbee), USB) for streaming and/or export of the signal data and/or for controlling the electronic unit (300), power source (*e.g*. rechargeable battery). The electronic unit (300) may comprise a processor configured for the extraction of signals (*e.g*. EEG, EMG, ECG). Alternatively or in addition, the processor configured for the generation of signals (*e.g*. tDCS). Operation of the electronic unit (300) may be controlled/co-ordinated by a processor. One or more visual output devices may be provided (*e.g*. LED indicators, matrix or alpha numeric display, LCD or LED screen). One or more audio output devices may be provided (*e.g*. sound transducer, loud speaker). One or more vibration output devices may be provided (*e.g*. motor, coil/magnet assembly). The (visual, audio and/or vibration) output device may indicate a correct and/or incorrect connection with the device (100). The (visual, audio and/or vibration) output device may indicate a quality of the contact between one or more of the electrodes and skin. One or more manual input devices may be provided (*e.g.* manual switch, touch switch, touch screen). An RFID reader may be provided with an RFID reader to read an RFID tag in the device (100); the electronic device may be configured to recognise an RDIF tag unique to the device (100) and to prevent re-use of the device (100) after a certain number of times (*e.g.* 1, 2, 3, 4, 5, less than 10).

The device (100) may be a one-time use device. An advantage of a one time use device is maintenance of sterility. The device (100) may be recyclable or reconditionable. The electronic unit (300) can be reused. There is an environmental advantage in reduction in chemical usage and water for cleaning. The device (100) may be disposable. The device (100) may have a limited-reusability (*e.g*. 2 to 5 times). The device (100) may have be washable, and/or sterilisable (*e.g.* with sterilizing reagent, by autoclaving, by UV light, etc).

The device (100) may have two or more different sizes, for instance an adult size, or children's size, or infant size.

The device (100) may comprise an identification tag (*e.g*. machine readable, QR code, RFID tag). The identification tag may be unique to the device. The unique identification tag prevents re-use of the device beyond its capacity for re-use.

The device (100) may indicate a correct and/or incorrect connection with the electronic unit (300). The device (100) may indicate a quality of the contact between one or more of the electrodes and skin. The indication may be visual (*e.g*. LED), audio, and/or vibration.

The main portion (140) may contain a measurement strip extending from the posterior end (10). The measurement strip contains a visually marked scale (*e.g*. printed, embossed, engraved, cut out) so that the size of the head can be measured. To take a measurement reading, the anterior end (12) of the main portion (140) is aligned with a bony landmark on the anterior side of the head (e.g. the nasion), and the position of a bony landmark on the posterior side of the head (*e.g.* inion) is read-off the scale on the measurement strip. The reading may be used to determine the tension applied to the main portion (140). For instance, which mounting aperture (152a, 152b, 152c) is to be selected corresponding to the head size (*e.g*. L,M,S), ensuring optimum electrode location. After use, the measurement strip may folded up, be detached, etc.

Provided is a system comprising the device (100) and the electronic unit (300).

Provided is a method of preparing the device (100) described herein, comprising:
- providing a one-piece outer inferior (14) layer of the planar support (120),
- providing a one-piece outer superior (12) layer of the planar support (120),
- optionally providing one or more inner layers containing a plurality of electrical conductors (156; 156,a; 156,b; 156,c; 156,d; 156,a'; 156,b'; 156,c'; 156,d'),
- attaching the plurality signal-conducting elements (148a to d) to the outer inferior (14) layer and optionally to one or more inner layers of the support (120),
- attaching the plurality of electrical connectors (144a to d) to the outer superior (16) layer and optionally to one or more inner layers of the support (120),
- joining together the one-piece outer inferior (14) layer, optional one or more inner layers, and one-piece outer superior (12) layer, thereby forming the device (100).

### Example

EEG data were collected using the presently-described device, wherein the bodily-worn support was disposed in a VR headset. A test group contained 20 healthy adults. The main body portion of the device contained 3 fixing elements on the posterior side (corresponding to sizes S, M and L), 3 signal conducting elements on the inferior side for attachment of dry EEG electrodes, and 1 electrode (pre-gelled) at the end of the side arm portion. The signal conducting elements were arranged to measure EEG data from Fz, Cz, and Pz locations on the scalp.

Head size from nasion to inion was measured for each subject, the used size of the fixing element was recorded, and electrode position along the midline was computed based on device characteristics. The results are shown in Table 1.

**Table 1**

| **Head size range [cm]** | **Size used** | **Deviation from true 10-20 electrode location [cm]** |
|---|---|---|
| 28-33 | S | 0.85 |
| 33-37 | M | 0.5 |
| 37-39 | L | 0.97 |

A tolerance of a 1 cm radius around the true 10-20 electrode location was implemented; within this tolerance, EEG signals are detectable and not significantly contaminated with EEG signals from other locations. The results show that, for different head sizes, the device allows for correct positioning of the electrodes for collection of EEG signals from the target location.

EEG signal quality was assessed: the quality of recorded signals based on signal to noise ratio, signal offset and signal characteristics such as amplitude and frequency content were evaluated, giving a quality indicator that ranges from 0 to 1. A quality indicator between 0.5 and 1 indicates that the quality of the recorded signal is sufficient for processing. Overall, 75% of collected data showed quality indicator above 0.5.

## Claims

1. Device (100) for placement of one or more skin-contacting electrodes on a subject (50) comprising:
- a planar support (120) having:
- a longitudinal main body portion (140) having a posterior end (10), an anterior end (12), an inferior side (14) and a superior side (16);
- a side arm portion (170) having a near end (18), a far end (20), an inferior side (14) and a superior side (16), wherein the side arm portion is joined at its near end (18) to the main body portion (140) towards the posterior end (10) of the main body portion (140);
- wherein a portion of the main body portion (140) contains a landing region (142, 142') for mechanical and electrical dismountable attachment of an electronic unit (300) on the superior side (16);
- wherein the main body portion (140) is longitudinally flexible so as to be able to conform to a shape of the midline of the head (52) of the subject (50);
- wherein the side arm portion (170) is at least longitudinally flexible so as to be able to bend towards the anterior end (12) of the longitudinal main body portion (140);
- a plurality of signal-conducting elements (148a to d) that are skin-contacting electrodes or mounts for dismountable attachment of skin-contacting electrodes;
wherein the inferior side (14) of the main body portion (140) comprises at least one signal-conducting element (148a to c); and
wherein the inferior side (14) of the side arm portion (170) comprises at least one signal-conducting element (148d).

2. The device (100) according to claim 1, wherein the support (120) is configured for adoption of a native conformation that is planar, and an active conformation in which the main body portion (140) is curved longitudinally to conform to a shape of midline (54) of the head (52) of the subject (50).

3. The device (100) according to claim 2, wherein the main body portion (140) is configured to adopt the active conformation by application of tension force (T) to the main body portion (140) between the posterior end (10) and the anterior end (12).

4. The device (100) according to claim 3, configured for attachment to a bodily-worn support (200), wherein the bodily-worn support (200) contains a supporting structure stably attachable to the subject's head, and configured for application of the tension force (T) to the main body portion (140).

5. The device (100) according to claim 4, wherein the main body portion (140) comprises a plurality of fixing elements (150) for dismountable attachment of the device (100) to the bodily-worn support (200).

6. The device (100) according to claim 5, wherein the plurality of fixing elements (150) are a combination of a plurality of posterior mounting apertures (152a, 152b, 152c) and an anterior mounting aperture (152d), configured to allow the application and adjustment of the tension force (T).

7. The device (100) according to any one of claims 1 to 6, wherein the landing region (142, 142') comprises a plurality of electrical connectors (144a to d; 144a' to d') on the superior side (16) for dismountable electrical attachment of the electronic unit (300) to the landing region (142).

8. The device (100) according to claim 7, wherein the landing region (142) comprises at least one mechanical connector (146a to d) for dismountable mechanical attachment of the electronic unit (300) to the landing region (142), optionally wherein each electrical connector (144a to d; 144a' to d') is also a mechanical connector (146a to d) for dismountable mechanical attachment of the electronic unit to the landing region (142).

9. The device (100) according to any one of claims 1 to 8, wherein the main body portion (140) is disposed with at least two discrete landing regions landing regions (142, 142'), one the landing region (142) positioned towards the posterior end (10) of the main body portion (140) and another landing region (142') positioned towards the anterior end (12) of the main body portion (140), and the electronic unit (300) is dismountably attachable to either (142, 142').

10. The device (100) according to claim 8 or 9, wherein the at least one mechanical connector (146a to d) is configured such that electronic unit (300) is dismountably attachable to the landing region (142, 142') in only one orientation.

11. The device (100) according to any one of claims 1 to 10, wherein at least a part of the planar support (120) is non-stretchable, preferably, wherein the planar support (120) contains a non-stretchable region spanning at least the signal-conducting element(s) (148a to c) of the main body portion (140).

12. The device (100) according to any one of claims 1 to 11, wherein the planar support (120) comprises:
- an outer inferior (14) layer that is outwardly exposed and skin-facing, wherein the main body portion (140) and the side arm portion (170) share a continuous outer inferior (14) layer,
- an outer superior (16) layer that is outwardly exposed and opposed to the outer inferior (14), wherein the main body portion (140) and the side arm portion (170) share a continuous outer superior (16) layer, and
- optionally at least one inner layer disposed between the outer superior (16) layer and the outer inferior (14) layer, wherein at least one inner layer is disposed with a plurality of electrical conductors for transmission of electrical signals between the plurality signal-conducting elements (148a to d) and the landing region (142, 142').

13. The device (100) according to any one of claims 1 to 12, containing at least three signal-conducting elements (148a to c) for conduction of EEG signals, and optionally EEG, EMG, ECG, tDCS signals, disposed spatially separated in a longitudinal direction on the main body portion (140), positioned to coincide with the Fz, Pz, and Cz positions on the head (52) of the subject (50).

14. The device (100) according to claim 13, wherein a distance between adjacent pairs of signal-conducting elements (148a-148b; 148b-148c) is fixed, is the same, and is between 45 and 105 mm.

15. The device (100) according to any one of claims 1 to 14, wherein the side arm portion (170) is configured for bending such that the signal-conducting element (148d) reaches the skin of the head (52) in a region posterior to the ear.
